# EUROPEAN PATENT SPECIFICATION

(11) **EP 3 367 995 B1**
(45) Date of publication and mention of the grant of the patent: **26.02.2020**
(21) Application number: 16791528.9
(22) Date of filing: 25.10.2016
(51) Int. Cl.: A61K 8/67, A61K 8/60, A61Q 19/02, A61Q 19/08, A61K 8/06, A61K 8/365

(54) **SKIN CARE COMPOSITION AND METHOD THEREOF**
HAUTPFLEGEZUSAMMENSETZUNG UND VERWENDUNGEN DAFÜR
COMPOSITION DE SOIN DE LA PEAU ET PROCÉDÉ ASSOCIÉ

(30) Priority: 30.10.2015 GB 201519184
(43) Date of publication of application: 05.09.2018
(73) Proprietor: The Boots Company PLC, Nottingham NG2 3AA (GB)
(72) Inventor: TOMLINSON, Paul James, Darley Abbey Derby DE22 1EX (GB); JOHNSON, Mark, Hucknall Nottingham NG15 8DQ (GB); HICKS, Jake, Thomas, Chilwell Nottingham NG9 6RR (GB)
(74) Representative: Agashi, Kapil
(86) International application number: PCT/EP2016/025124
(87) International publication number: WO 2017/071820

(56) References cited:
- WO-A1-2015/002091
- CN-A- 103 211 718
- JP-A- H01 213 212
- US-A1- 2008 287 533
- DATABASE GNPD [Online] MINTEL; 1 September 2015 (2015-09-01), XP002764865, Database accession no. 3368823
- DATABASE GNPD [Online] MINTEL; 1 August 2014 (2014-08-01), XP002764866, Database accession no. 2576871
- DATABASE GNPD [Online] MINTEL; 1 December 2013 (2013-12-01), XP002764867, Database accession no. 2250379
- DATABASE GNPD [Online] MINTEL; XP002764868, Database accession no. 1735496
- DATABASE GNPD [Online] MINTEL; 1 July 2011 (2011-07-01), XP002764869, Database accession no. 1591480

## Description

### TECHNICAL FIELD

The disclosed technology relates to a composition comprising an O-substituted ascorbic acid or derivative thereof; and a gluconolactone. The disclosed technology further relates to the use and method of improving skin condition.

### BACKGROUND OF THE INVENTION

Vitamin C and gluconolactone (often referred to as gluconodelta-lactone) are various derivatives known for use in cosmetics.

Gluconolactone is believed to be an acidic compound that is gentle on the skin and provides skin care compositions with anti-aging performance such as reduced wrinkles or reduced fine lines. Gluconolactone has a larger molecular structure than many alpha- and/or beta- hydroxycarboylic acids known in skin care compositions such as lactic acid, ascorbic acid, or glycolic acid. The effectiveness of gluconolactone is believed to be optimal at lower pH conditions.

Vitamin C and various derivatives based upon ascorbic acid, is believed to enhance collagen synthesis, or as an antioxidant and has been used in compositions for anti-aging (through reduced wrinkle, fine lines or improving skin firmness), and providing brighter/radiant skin. Compositions containing Vitamin C or its derivatives have been suggested to reduce the appearance of brown spots and other types of sun damage, and to improve skin's natural healing response. It is believed that the instability of Vitamin C in topical compositions may be due to its ease of oxidation by oxygen, light, alkaline conditions, some metals and elevated temperature. As a result of the instability of Vitamin C in topical compositions it is believed that optimal formulations have a low pH. In order to stabilise Vitamin C, various attempts have been made in skin care compositions as summarised below in aqueous and non-aqueous media.

US 2,400,171 (Ruskin, published 14 May 1946) relates to the conversion of ascorbic acid to its calcium or zinc salt to maintain stable aqueous solutions.

US 6,146,664 (Siddiqui, published 14 November 2000) relates to a composition containing ascorbic acid in a nonaqueous or substantially anhydrous silicone vehicle.

US 8,022,090 (Choi et al., published 20 September 2011) relates to a cationic material and anionic material as a primary stabilizing agent of the vitamin C; and a caffeic acid derivative as a secondary stabilizing agent of the vitamin C.

US2007196310 (Mary Kay, published 21 February 2007) relates to a non-aqueous composition comprising:(a) an ascorbic acid; (b) a silicone containing compound in an amount of less than 50% by weight of the total composition; and(c) an essential oil, wherein at least 50% of the initial amount of the ascorbic acid in the composition remains stable when the composition is stored for at least 1 month at room temperature.

US 2014/0155633 (Lin-Chao et al, published 5 June 2014) relates to compositions for stabilizing ascorbic acid derivatives by defining an alkyl-substituted ascorbic acid.

US 7,741,496 (Wei-Chuan et al., published 22 June 2010) and US 2011/02811943 (Pei Miu et al., published 17 November 2011) both relate to an ascorbic acid derivative that combines one or two hydrophilic head groups connected by a hydrophobic spacer.

US 6,110,476 (Nguyen et al., published 29 August 2000) relates to a system based upon phosphonic acid derivative and metabisulfate to stabilise ascorbic acid. US 2008/0253982 (Shibayama, published 16 October 2008) discloses stable ascorbic acid phosphate esters.

US 6,162,419 (Perricone et al., published 19 December 2000), US 6,087,393 (Mathur, published 7 July 2000), US 8,053,469 (8 November 2011), US 5,736,567 (Cantin et al., published 7 April 1998), and US 5,140,043 (Darr et al., published 18 August 1992) all relate to a stabilized system of ascorbic acid in a variety of glycol compounds.

US 6,010,706 (Candau et al., published 4 January 2000) relates to a container for stabilising ascorbic acid in a hydrophilic carrier which are packaged separately but mixed together for use.

The patents mentioned above provide a considerable number of options for stabilising ascorbic acid or various derivatives by using a significant number of different chemical approaches, a container and in oil-in-water as well as water-in-oil compositions.

None of the stabilisation approaches considered by the prior art above disclose the use of the derivative in combination with gluconolactone, or derivatives thereof.

US 6,036,963 (Weinkauf et al, published 14 March 2000) relates to oil-in-water cosmetic skin care emulsion composition comprising a hydroxy acid, gluconolactone or glucarolactone in an amount of from about 3% to about 12%, and a cosmetically acceptable vehicle.

US 2008/0287533 (Gupta, published 20 November 2008) relates to esters of ascorbic acid with natural sugar lactones, and one of the sugar lactones disclosed is gluconolactone. US 2008/0287533 thus teaches combining both the ascorbic acid and the lactone to form a single molecule.

### SUMMARY OF THE INVENTION

It would be advantageous to prepare a composition comprising gluconolactone and a form of Vitamin C to improve skin care condition (may also be referred to as skin condition). Improved skin care may include decrease or prevent at least one of the following improving skin exfoliation, moisturisation, forming wrinkles or fine lines, skin sagging, or hyperpigmentation (such as solar lentigines), or increase skin firmness or skin laxity. However, due to the difference in the believed optimum functional conditions outlined above it would be advantageous to have a composition having improved stability due to enhanced stability of the form of Vitamin C in a composition comprising gluconolactone.

The person skilled in the art knows that gluconolactone in an aqueous based system may be partially hydrolysed to gluconic acid with the balance between the lactone form and the acid form. This is a well-known chemical equilibrium. As a result reference to gluconolactone herein is intended to also encompass both gluconolactone, and any equilibrium products including gluconic acid.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of and "consisting of," where "consisting of excludes any element or step not specified and "consisting essentially of permits the inclusion of additional un-recited elements or steps that do not materially affect the basic, essential and novel characteristics of the composition, method or use under consideration.

Unless otherwise indicated treat rates are on a weight basis relative to the total composition disclosed herein.

The disclosure relates to a cosmetic composition comprising:
0,001 to 10% wt of an O-substituted ascorbic acid represented by the formula: 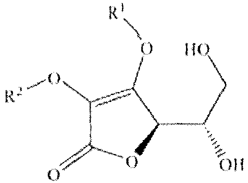 wherein R¹ is C1-20 alkyl or C3-20 cycloalkyl and R² is H; and
0.01 to 10 wt % gluconolactone,
wherein the composition comprises an aqueous phase present at up to 95 wt %, or up to 90 wt % of the composition.

In one embodiment the disclosed technology relates to a cosmetic composition comprising:
0,001 to 10% wt of an O-substituted ascorbic acid represented by the formula: 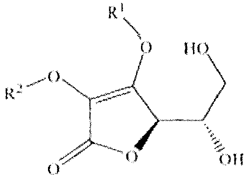 wherein R¹ is C1-20 alkyl or C3-20 cycloalkyl and R² is H; and
0.01 to 10 wt % gluconolactone,
wherein the composition is an emulsion comprising an oil phase and an aqueous phase present at up to 90 wt % of the composition.

In one embodiment the disclosed technology relates to a cosmetic composition comprising:
0,001 to 10% wt of an O-substituted ascorbic acid represented by the formula: 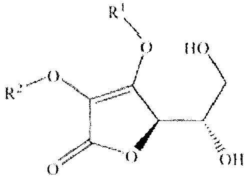 wherein R¹ is C1-20 alkyl or C3-20 cycloalkyl and R² is H; and
0.01 to 10 wt % gluconolactone,
wherein the composition is a gel and the aqueous phase present at up to 95 wt % of the composition.

In one embodiment the disclosed technology relates to a cosmetic composition comprising 0.001 to 5 wt % of an O-substituted ascorbic acid defined above, and 0.01 to 5 wt % of gluconolactone, wherein the composition comprises an aqueous phase present at up to 95 wt %, or up to 90 wt % of the composition.

In one embodiment the disclosed technology relates to a cosmetic composition comprising 0.01 to 3 wt % of an O-substituted ascorbic acid defined above, and 0.1 to 2.5 wt % of gluconolactone, wherein the composition comprises an aqueous phase present at up to 95 wt %, or up to 90 wt % of the composition.

In one embodiment the disclosed technology relates to a cosmetic composition comprising 0.1 to 2 wt % of an O-substituted ascorbic acid defined above, and 0.5 to 2 wt % of gluconolactone, wherein the composition comprises an aqueous phase present at up to 95 wt %, or up to 90 wt % of the composition.

The gluconolactone disclosed herein may include gluconodeltalactone, or glucono-1,5-lactone (typically gluconodeltalactone).

Typically the composition comprises a higher concentration of the gluconolactone than the O-substituted ascorbic acid defined above.

The composition may be in the form of a gel, cream, lotion or serum, typically a cream, serum, lotion. In one embodiment the composition may be a serum.

The composition may be an emulsion or gel.

When the composition is an emulsion, the composition may be an emulsion comprising an oil phase and an aqueous phase present at up to 90 wt % of the composition.

When the composition is a gel, the composition further comprises a thickener.

In one embodiment the disclosed technology relate to the cosmetic use of a cosmetic composition disclosed herein (typically a skin care composition).

In one embodiment the disclosed technology relate to a method of improving skin care comprising supplying to skin the composition disclosed herein. Improving skin care may include improving (i) skin exfoliation, or moisturisation, or (ii) decreasing or preventing at least one of the following, forming wrinkles or fine lines, skin sagging, or hyperpigmentation (such as solar lentigines), or (iii) increasing skin firmness or skin laxity).

In one embodiment the disclosed technology relate to the use of the composition disclosed herein to improve skin care. Improved skin care may include improving (i) skin exfoliation, or moisturisation, or (ii) decreasing or preventing at least one of the following, forming wrinkles or fine lines, skin sagging, or hyperpigmentation (such as solar lentigines), or (iii) increasing skin firmness or skin laxity).

The use and method disclosed herein are known to the skilled person as not encompassing therapeutic or medical treatment i.e., the disclosed use or method relate to a non-therapeutic use or method.

Typically skin is a mammalian skin such as human skin.

### DETAILED DESCRIPTION OF THE INVENTION

The disclosed technology provides a composition, methods and uses as disclosed above.

### O-Substituted Ascorbic Acid

The O-substituted ascorbic acid defined above is O-alkyl ascorbic acid.

The alkyl may be acyclic or cyclic, typically acyclic. The acyclic group may be linear or branched, typically linear.

O-substituted ascorbic acids and derivatives thereof are known in the art, and described in EP Patent application EP2722043 A1, and US 2014/0155633 (both Lin et al., Applicant Corum).

The O-substituted ascorbic acid defined above has a substituted group that may be hydrocarbon in nature i.e., composed on carbon and hydrogen. R¹ is C1-20 alkyl or C3-20 cycloalkyl.

In one embodiment the O-substituted ascorbic acid may be 3-alkyl ascorbic acid, or mixtures thereof. Typically the alkyl group may be a C1-20, or C1-10, or C2-8, or C2-4.

Typically the O-substituted ascorbic acid may be 3-ethyl ascorbic acid.

The O-substituted ascorbic acid may be present at 0.001 to 5 wt %, or 0.01 to 3 wt %, or 0.1 to 2 wt % of the composition.

### Other Ingredients

The composition disclosed herein may optionally further comprise other ingredients. The other ingredients include Hibiscus, a peptide, a matrix metalloproteinase inhibitor, a whitening agent, a skin conditioning agent, a salicylic acid compound, a sunscreen agent, preservatives thickeners, viscosity modifying agents, and/or gelling agents sequestering agents, wax, diluents, carriers, propellants perfumes, or pH adjusting agents.

In one embodiment the composition disclosed herein further comprises one or more of Hibiscus, a peptide, a matrix metalloproteinase inhibitor, a whitening agent.

The Hibiscus may be Hibiscus sabdariffa, Hibiscus rosa sinensis or Hibiscus Abelmoschus. All three Hibiscus plants are known to form extracts used in cosmetic compositions. The Hibiscus may be in the form of an extract.

Peptides are defined as compounds comprising an uninterrupted sequence of amino acids. Typically the peptides are of natural origin. A dipeptide comprises an uninterrupted sequence of two amino acids. Amino acids, as employed herein, include and encompass all of the naturally occurring amino acids, either in D or L configuration. Amino acids are commonly indicated with reference to the conventional three letter code and the sequence is read from left to right. The composition of the disclosed technology may comprise a dipeptide chosen from acetyl dipeptide 1 cetyl ester, acetyl dipeptide 3 aminohexanoate, azelaoyl bisdipeptide 10, coumaroyl dipeptide 3, dicetyl dipeptide 9, dipeptide diamino butyroyl benzylamide diacetate, dipeptide 1, dipeptide 10, dipeptide 11, dipeptide 12, dipeptide 15, dipeptide 16, dipeptide 17, dipeptide 18, dipeptide 19, dipeptide 2, dipeptide 20, dipeptide 3, dipeptide 4, dipeptide 5, dipeptide 6, dipeptide 7, dipeptide 8, dipeptide 8 HCL, dipeptide 9, hexanoyl dipeptide 3 norleucine acetate, methyl undecylenoyl dipeptide 16, nicotinoyl dipeptide 22, nicotinoyl dipeptide 23, nicotinoyl dipeptide 24, nicotinoyl dipeptide 26, oleoyl dipeptide 15, palmitoyl dipeptide 10, palmitoyl dipeptide 13, palmitoyl dipeptide17, palmitoyl dipeptide 5 diaminobutyroyl hydroxythreonine, palmitoyl dipeptide 5 diaminohydroxybutyrate, palmitoyl dipeptide 7 and mixtures thereof.

In one embodiment the composition of the disclosed technology may comprise a tripeptide, or mixtures thereof. The tripeptide may be naturally occurring or of synthetic origin. Suitable tripeptide compounds include tripeptide 1, 2, 3, 4, 5, 6, 7, 8, 9, 10,11, 12, 13, 14, 15, 16, 17, 18, 19, 20, 21, 22, 23, 24, 25, 26, 27, 28 ,29, 30, 31, 32, 33, 34, 35, 36 ,37, 38, 39, 40, 41, 42, 43, 44, 45, 46, derivatives thereof, and mixtures thereof. The tripeptide comprise one or more His-based tripeptides.

The compositions of the disclosed technology may further comprise a tetrapeptide. The tetrapeptide may be one or more rigin-based tetrapeptides, one or more ALAMCAT-tetrapeptides or mixtures thereof. The tetrapeptide may be naturally occurring or of synthetic origin. Suitable tetrapeptides for use in the present composition include those chosen from tetrapeptide 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 34, 35, derivatives thereof and mixtures thereof.

Rigin-based tetrapeptides of the disclosed technology may be based on the structure Gly-Gln-Pro-Arg (Rigin) and include its analogs and derivatives thereof. Rigin is a typical tetrapeptide.

The compositions of the disclosed technology may further comprise a pentapeptide, derivatives of thereof, and mixtures thereof. As used herein, "pentapeptide" refers to both the naturally occurring pentapeptide and synthesized pentapeptide. Also useful herein are naturally occurring and commercially available compositions that comprise pentapeptides. Suitable pentapeptides are those chosen from pentapeptide 1, 4, 5, 6, 7, 8, 9, 10, 11, 12, 13, 14, 15, 16, 17, 18, 19, 21, 22, 23, 24, 25, 26, 28, 29, 30, 31, 33, 34, 35, 36, 38, 39, derivatives thereof and mixtures thereof.

The peptide when present in the composition disclosed herein may be present at 0 or 0.01% to 20%, or 0.05% to 15%, or 0.05 to 10 wt % of the composition.

### Matrix Metalloproteinase Inhibitor (MMPi)

The term "matrix metalloproteinase inhibitor" relates to all molecule and/or plant or bacterial extracts having an inhibitory activity on at least one of the matrix metalloproteinases expressed, synthetized, or activated by or in the skin. The family of the matrix metalloproteinases is formed of several well-defined groups on the basis of their resemblance regarding structure and substrate specificity (Woessner J. F., Faseb Journal, vol. 5, 1991, 2145).

The MMPi may be present at a level of from 0 or 0.01% to 10%, or 0.1% to 5% or 0.25% to 2.5%, or 0.5% to 1% by weight of the composition.

### Whitening Agent

In one embodiment the composition disclosed herein further comprises a whitening/lightening agent.

When the composition further comprises the whitening/lightening agent it may be present at 0 or 0.001 to 10 wt %, or 0.01 to 5 wt %, or 0.1 to 2 wt %, or 0.2 to 1 wt % of the composition. For example the whitening/lightening agent may be present at 0 or 0.001% to 3 wt %, or 0.01 to 2 wt%, or 0.05 to 1 wt %, or 0.1% to 0.5 wt % of the composition.

The whitening/lightening may include at least one of the following ingredients: Emblica, Mulberry leaf extract, mangostin, Sophora, a flavonoid, hydroxyphenoxy propionic acid and dimethylmethoxy chromanol.

In one embodiment the whitening/lightening agent may be a mixture of ingredients chosen from:
Emblica, and
an antioxidant chosen from Mulberry leaf extract, mangostin, Sophora, a flavonoid, hydroxyphenoxy propionic acid and a chromane.

In one embodiment the whitening/lightening agent may be a mixture of ingredients chosen from: Emblica and Sophora, optionally in the presence of Mulberry leaf extract. Typically the Mulberry leaf extract may be present.

The Emblica may be Emblica officinalis, typically comprising over 40% by weight (typically 50-80 wt %) of Emblicanin A. Emblicanin B, Pedunculagin and Punigluconin, and not more than about 1% by weight of flavonoids.

The Emblica may be phyllanthus Emblica.

The Sophora may be an extract of a small tree, and shrub in the pea family Fabaceae.

Sophora Angustifolia Root Extract a Sophora Alopecuroides Extract, sophora japonica extract,
The Sophora Angustifolia Root Extract may be an extract of the roots of the Chinese Sophora, Sophora angustifolia, or Leguminosae.

In one embodiment Sophora is derived from Sophora Angustifolia Root Extract.

Typically The Emblica may be phyllanthus Emblica and Sophora is derived from Sophora Angustifolia Root Extract.

The flavonoid species is believed to have antioxidant performance, and be an antioxidant plant polyphenolic agent. By the term antioxidant plant polyphenolic agent we mean a plant extract, or derivative thereof, comprising flavonoid species, including flavones, flavonols, flavanones, flavanols anthrocyanidins and isoflavonoids; phenolic acid species; stilbenes; lignans and mixtures thereof, which provide an antioxidant benefit. Antioxidant benefit is measured using the total antioxidant capacity (TAC) assay described herein. Plants provide a rich source of polyphenolic agents, and are therefore an efficient source of said antioxidants. Similar actives may be prepared synthetically and as such are analogues of said plant polyphenolic agents.

Antioxidant polyphenolic agents may include extracts from plants chosen from Mulberry (e.g. morus alba), Ginseng (e.g. Panax ginseng), Raspberry, Oregano (e.g. origanum vulgare), Green tea (e.g. green leaves of camellia sinensis), White tea (e.g. camellia sinensis), Blueberry extract (e.g. vaccinium cyanococcus), French maritime pine bark (e.g. pinus pinaster, sold under the tradename Pycnogenol), Rosemary (e.g. rosmarinus officialis), Grape, including grape seed (e.g. vitis vinifera), Fennel (e.g. foeniculi fructus), Caragana sinica, Marjoram (e.g. origanum majorana), Crocus (e.g. crocus sativus), Apple (e.g. malus domestica), Coffee, Green coffee, Cherry (e.g. prunus avium), Snow algae (e.g. chlamydomonas nivalis), Emblica (e.g. Phyllanthus emblica), Gingko (e.g. Gingko biloba), Moringa (e.g. moringa oleilera), Ginger (e.g. zingiberaceae), Magnolia (e.g. magnolioideae virginiana), French saffron, Edelweiss (e.g. leontopodium alpinium), White lotus (e.g. nymphaea alba), Turmeric root, Marshmallow (e.g. althaea officianlis), Burdock (e.g. arctium lappa), Bilberry (e.g. vaccinium myrtillus), Cranberry (e.g. vaccinium oxycoccus), Pomegranate nectar (e.g. Punica granatum), Sage (e.g. salvia officinalis), Thyme (e.g. thymus vulgaris), Sunflower (e.g. helianthus annuus), wild carrot (e.g. daucus carota), Hop (e.g. humulus lupulus), Witch Hazel (e.g. hamamelis), Oak (e.g. Quercus), Camellia (e.g. theacea), Red clover (e.g. tritolium pratense), Flax (e.g. linium usitatissimum), lemon (e.g. citrus limon), birch (e.g. betula), cornflower, (e.g. centaurea cyanus), geranium, polygonum, soy (e.g. glycine max), and mixtures thereof.

In one embodiment the antioxidant polypenolic agent may be an extract from a plant chosen from mulberry, ginseng, grape, oregano, grape, sage, sunflower, maritime pine bark, rosemary, marjoram, crocus, french saffron, wild carrot, hop, coffee, green coffee, witch hazel, oak, camellia, red clover, flax, ginger, magnolia, edelweiss, burdock and mixtures thereof.

Active polyphenolic species sourced from the above list of plants include those chosen from apigenin, luteolin, quercetin, kaempferol, naringenin, hesperetin, catechin, gallocatechin, cyaniding, pelargonidin, daidzein, genistein, caffeic acid, chlorogenic acid, romsmarinic acid, gallic acid, resveratrol, ferulic acid, epigallocatechin gallate, piceatannol, secoisolariciresinol, isotaxiresinol, Miyabenol c, Luteolin and mixtures thereof.

The amounts of antioxidant plant polyphenolic agents used in the present invention are expressed as dry weights of the extract, as understood by a man skilled in the art. The antioxidant plant polyphenolic agent (plant extract) may be present at 0.005 to 10 wt, or 0.01 to 7 wt %, or 0.01 to 5 wt % of the composition.

When present the chromane may be chosen from: methyl, di-, tri- and tetra- C1-C6 alkyl, C1-C6 alkoxy chromanol; pentamethyl chromanol, methyl, di, tri and tetra C1-C6 alkyl, C1-C6 alkoxy chromanyl C14-C20 ester and mixtures thereof.

Typically the chromane may be chosen dimethyl methoxy chromanol, tetramethyl methoxy chromanol, pentamethyl chromanol, dimethyl methoxy chomanyl palmitate, dialkyl methoxy chomanyl myristate, dimethyl methoxy chromanyl stearate, dimethyl methoxy chomanyl oleate, dimethyl methoxy chomanyl linoleate and mixtures thereof.

In one embodiment the chromane may be dimethyl methoxy chromanol (commercially available under the tradename Lipochroman 6 as sold by Lipotec).

### Skin Conditioning Agent

The composition of the present invention may optionally comprise a skin conditioning agent. The skin conditioning agents may be chosen from humectants, emollients, moisturisers, or mixtures thereof. Where present, the skin conditioning agent may be present from 0 or 0.01% to 20%, or 0.1% to 10%, or 0.5% to 7% by weight of the composition.

The skin conditioning agents may be chosen from guanidine, urea, glycolic acid and glycolate salts, salicylic acid, lactic acid and lactate salts, aloe vera, shea butter, polyhydroxy alcohols, such as sorbitol, mannitol, xylitol, erythritol, glycerol, hexanetriol, butanitriol, (di) propylene glycol, butylene glycol, hexylene glycol, polyethylene glycol, sugars (e.g. fructose, glucose, xylose, honey, mannose, xylose), gluconodeltalactone, and starches and their derivatives, pyrrolidone, carboxylic acid, hyaluronic acid and salts thereof, lactamide monoethanolamine, acetamide monoethanolamine, panthenol, allantoin and mixtures thereof.

Typically the skin conditioning agent is chosen from glycerine, arabinoglactan, butylene glycol, hyaluronic acid, shea butter, propylene glycol, ethylhexyl glycerine, hyaluronate and mixtures thereof.

### Salicylic Acid Compound

The compositions disclosed herein may optionally comprise a salicylic acid compound, its esters, its salts, or combinations thereof. In one embodiment of the composition disclosed herein comprises a salicylic acid compound at 0 or 0.0001% to 25%, or 0.001% to 15%, or 0.01% to 10%, or 0.1% to 5%, and or 0.01% to 2%, by weight of the composition, of salicylic acid. In one embodiment the salicylic acid compound is salicylic acid.

### Sunscreen

The composition of the present invention may optionally comprise a sunscreen component. The sunscreen may comprise organic or inorganic sun filters or a combination of the two. Suitable inorganic sunfilters include those chosen from microfine titanium dioxide, and microfine zinc oxide, and mixtures thereof.

Suitable organic sunscreens include those chosen from: a) p-aminobenzoic acids, their esters and derivatives (for example, 2-ethylhexyl p-dimethylaminobenzoate), b) methoxycinnamate esters (for example, 2-ethylhexyl p-methoxycinnamate, 2-ethoxyethyl p-methoxycinnamate or a, p-di- (p-methoxycinnamoyl)-a'- (2ethylhexanoyl)-glycerin, c) benzophenones (for example oxybenzone), d) dibenzoylmethanes such as 4- (tert-butyl)-4'-methoxydibenzoylmethane, e) 2-phenylbenzimidazole-5 sulfonic acid and its salts, f) alkyl-ss, ss-diphenylacrylates for example alkyl a-cyano-ss, ss-diphenylacrylates such as octocrylene, g) triazines such as 2,4,6-trianilino- (p-carbo-2-ethyl-hexyl-1-oxi)-1, 3,5 triazine, h) camphor derivatives such as methylbenzylidene camphor and i) mixtures thereof. Other sunscreen ingredients include those chosen from homosalate, Ethylhexyl salicylate, Diethylhexylbutamido triazone, Bis-ethylhexyloxyphenol methoxyphenyl triazine, Diethylamino hydroxybenzoyl hexyl benzoate, Butyl methoxydibenzoylmethane, Methylene bis-benzotriazoyl tetramethylbutylphenol, Polysilicone-15 and mixtures thereof. A sunscreening agent may be present from 0 to 10 %, or 0.1 to 10% by weight of the composition.

### Other Optional Ingredients

Preservatives may be added to the composition such as benzoic acid, sodium benzoate, sorbic acid, potassium sorbate, 2-bromo2-nitropropane-1,3-diol (bronopol, which is available commercially under the trade name Myacide ®, benzyl alcohol, diazolidinyl urea, imidazolidinyl urea, methyl paraben, phenoxyethanol, ethyl paraben, propyl paraben, sodium methyl paraben, sodium dehydroacetate, polyhexamethylenebiguanide hydrochloride, isothiazolone and sodium propyl paraben and mixtures thereof, suitably in an amount of from 0.01% to 10% by weight of the composition.

Sequestering agents may be added to the composition, such as ethylenediamine tetraacetic acid and salts thereof, typically in an amount of from 0 or 0.005 to 0.5 wt % of the composition.

The composition may include waxes such as cocoa butter suitably in an amount of from 0.1 to 10 wt % of the composition.

The composition may also comprise suitable, cosmetically acceptable diluents, carriers and/or propellants such as dimethyl ether. The composition may also include pearlising agents such as stearic monoethanolamide and/or mica, suitably in an amount of from 0 or 0.01% to 10% by weight of the composition.

Perfumes may be added suitably in an amount of from 0 or 0.01% to 2% by weight of the composition, as may water soluble dyes such as tartrazine, suitably in an amount of from a trace amount such as 0 or 1 x 10⁻⁵ % to 0.1 % by weight of the composition.

The composition may also include pH adjusting agents such as sodium hydroxide, amino methyl propanol, triethanolamine, suitably in an amount of from 0 or 0.01 % to 10% by weight of the composition. The composition may be buffered by means well known in the art, for example by use of buffer systems comprising succinic acid, citric acid, lactic acid, and acceptable salts thereof, phosphoric acid, mono-or disodium phosphate and sodium carbonate. Suitably, the composition may have a pH between 3 and 10, between 4 and 8, or between 4.5 and 6.5.

In one embodiment the composition of the disclosed technology does not contain a ascorbic acid derivative chosen from sodium ascorbyl phosphate, ascorbyl glucoside, L-ascorbic acid, ascorbyl palmitate, retinyl ascorbate, tetrahexyldecyl ascorbate, or magnesium ascorbyl phosphate.

In one embodiment the composition of the disclosed technology does not include MMP compounds that comprise one hydroxyaryl or polyhydroxyaryl compound, or cyclic compounds having a cyclic group based upon a compound comprising a pyran, a lactam, or a piperidine constituent.

A thickener, viscosity modifying agent and/or gelling agent may be added to the composition, such as acrylic acid polymers e. g. available commercially under the trade name CarbopolOO or Ultrez® (both Lubrizol), or a taurate copolymer such as acryloyl methyl taurate-vinylpyrrolidone copolymers, or hydroxyethylacrylate/sodium acryloyldimethyl taurate copolymers, or modified celluloses e. g. hydroxyethylcellulose available commercially under the trade name Natrosol® (Hercules) or hydroxypropylmethyl cellulose, amine oxides, block polymers of ethylene oxide and propylene oxide (for example, those available from BASF Wyandotte under the trade name"Pluronic"®), PVM, MA, or a decadiene crosspolymer (available under the trade name Stabilez® 60), ethoxylated fatty alcohols, salt (magnesium chloride, sodium chloride), Aristoflex®AVC (Clariant), phthalic acid amide, xanthan gum, starch, or modified starch (such as a metal salt of starch e.g., aluminum salt of the reaction product of 1-octenylsuccinic anhydride with starch), sodium polyacrylate, polyvinyl alcohols, fatty alcohols and alkyl galactmanans available under the trade name N-Hance® from Hercules.

### Gel

When in the form of a gel, the composition disclosed herein may contain 0.1 to 10 wt %, or 0.5 to 5 wt %, or 0.5 to 3 wt % of a thickener such as a viscosity modifying agent and/or gelling agent, and may be typically a polymeric thickener.

The gel may comprise:
50 to 95 wt % water and 0.1 to 10 wt % of a thickener, or
60 to 95 wt % water, and 0.5 to 5 wt % of a thickener, or
70 to 90 wt % of water; and 0.5 to 3 wt % of a thickener.

### Emulsion

The emulsion disclosed herein may be a water-in-oil, oil-in-water, or water-in-silicone composition, typically oil-in-water composition.

The emulsion may be an oil-in-water emulsion, or a water-in-silicone oil emulsion, typically an oil-in-water emulsion.

The emulsion may comprise an oil phase and have an aqueous phase content of 30 to 85 wt %, or 50 to 80 wt %, or 60 to 75 wt % of the composition.

The aqueous phase may contain water present at 40 to 80 wt %, or 50 to 75 wt %, or 60 to 75 wt % of the composition.

The emulsion may comprise an oil phase having 15 to 70 wt %, or 30 to 50 wt %, or 25 to 40 wt % of the composition.

The emulsion may be an oil-in-water composition comprising 15 to 70 wt % of an oil phase; and 30 to 85 wt % of an aqueous phase.

The emulsion may be an oil-in-water composition comprising 25 to 40 wt % of an oil phase; and 60 to 75 wt % of an aqueous phase.

The emulsion may be in the form of a water-in-silicone emulsion, and the water phase may be present at 30 to 85 wt % of an aqueous phase; and silicone present at 15 to 70 wt % of a silicone phase.

The emulsion may be in the form of a water-in-silicone emulsion, and the water phase may be present at 60 to 75 wt % of an aqueous phase; and silicone present at 25 to 40 wt % of a silicone phase.

If the composition disclosed herein is in the form of a water-in-silicone composition the oil phase may be provided by any suitable silicate, dimethiconols, silicone elastomer and mixtures thereof (typically a silicone elastomer).

Typically the silicone oil phase may be formed from an organopolysiloxane. The organopolysiloxane may be chosen from one or more of a polyalkylsiloxane, alkyl substituted dimethicone, cyclomethicone, trimethylsiloxysilicate. dimethiconol, polyalkylaryl siloxane, and mixtures thereof. The polyalkylsiloxane may be for example a cyclomethicone, or dimethicone, typically a dimethicone.

A water-in-silicone composition disclosed herein may include an emulsifying crosslinked organopolysiloxane elastomer, a non-emulsifying crosslinked organopolysiloxane elastomer, or a mixture thereof. The term "non-emulsifying," as used herein, defines crosslinked organopolysiloxane elastomers from which polyoxyalkylene units are absent. The elastomers may include dimethyl polysiloxanes crosslinked by Si-H sites on a molecularly spherical MQ resin. Emulsifying crosslinked organopolysiloxane elastomers include the crosslinked polymers described in US Patents 5,412,004; 5,837,793; and 5,811,487. The emulsifying elastomer comprised of dimethicone copolyol crosspolymer (and) dimethicone is commercially available from Shin Etsu under the trade name KSG-21.

The non-emulsifying elastomers may include dimethicone crosspolymers. Such dimethicone crosspolymers are supplied by a variety of suppliers including Dow Corning (EL9240). Other dimethicones crosspolymer are available from General Electric (SFE 839), Shin Etsu (KSG-15, 16, 18 [dimethicone/phenyl vinyl dimethicone crosspolymer]), and Grant Industries (GRANSIL™ line of elastomers). Cross-linked organopolysiloxane elastomers useful in the present invention and processes for making them are further described in US Patents 4,970,252; 5,760,116; and 5,654,362. Commercially available elastomers typical for use herein are Dow Coming's 9040 silicone elastomer blend, Shin Etsu's KSG-21, and mixtures thereof.

An oil-in-water or water-in-oil emulsion may comprise an organic oil. The organic oil may be volatile or non-voaltile. The organic oil may include a diluent, a solvent, a polyolefin polymer, or an ester oil.

The term "ester oil" means an oil that is liquid at room temperature (25 °C.) comprising at least one ester functional group. The ester oil used herein is chosen, for example, from monoesters.

The ester oil may, for example, be chosen from the monoesters of formula R¹COOR² wherein R¹ may be selected from linear and branched hydrocarbon-based chains comprising from 4 to 30, or 6 to 24, or 7 to 20 carbon atoms carbon atoms, and R² may be chosen from branched hydrocarbon-based chains comprising from 3 to 40 carbon atoms, such as from 10 to 30 carbon atoms and further such as from 16 to 26 carbon atoms.

Examples of the ester oils that may be mentioned include isodecyl neopentanoate; isocetyl octanoate; isononyl isononanoate, isodecyl isononanoate, tridecyl isononanoate; hexyl laurate, 2-hexyldecyl laurate; isopropyl myristate, isocetyl myristate, isotridecyl myristate, 2-octyldodecyl myristate; isopropyl palmitate, 2-ethylhexyl palmitate, isooctyl palmitate, isocetyl palmitate, isodecyl palmitate, isostearyl palmitate, 2-octyldecyl palmitate; isopropyl isostearate, 2-octyldodecyl stearate, isostearyl isostearate, and 2-octyldodecyl erucate.

The ester oil may be present in the emulsion disclosed herein in an amount ranging, for example, from 0 to 20 wt %, or 0.1 to 15 wt %, or 1 to 10 wt % of the composition.

### EXAMPLES

A series of compositions are prepared using the following general procedure. A percentage of water is added to a vessel, and a percentage of gluconolactone is added to form a mixture. The mixture is then stirred for approximately 30 minutes. A percentage of Vitamin C, or a derivative thereof is added to the vessel and stirred until it is dissolved. The pH is adjusted to 4.6 ± 0.1 using a 50 w/w solution of either potassium hydroxide or citric acid powder. The compositions prepared are:
Comparative Example 1 (CE1): a composition containing 0.5 wt % of vitamin C (ascorbic acid), and 1.5 wt % of gluconolactone.
Comparative Example 2 (CE2): a composition containing 0.5 wt % of sodium ascorbyl phosphate, and 1.5 wt % of gluconolactone.
Comparative Example 3 (CE3): a composition containing 0.5 wt % of ascorbyl glucoside, and 1.5 wt % of gluconolactone.
Inventive Example 1 (IE1): a composition containing 0.5 wt % of 3-ethyl ascorbic acid, and 1.5 wt % of gluconolactone.
Comparative Example 4 (CE4): a composition containing 1 wt % of vitamin C (ascorbic acid), and 1.5 wt % of gluconolactone.
Comparative Example 5 (CE5): a composition containing 1 wt % of sodium ascorbyl phosphate, and 1.5 wt % of gluconolactone.
Comparative Example 6 (CE6): a composition containing 1 wt % of ascorbyl glucoside, and 1.5 wt % of gluconolactone.
Inventive Example 2 (IE2): a composition containing 1 wt % of 3-ethyl ascorbic acid, and 1.5 wt % of gluconolactone.
Comparative Example 7 (CE7): a composition containing 0.5 wt % of vitamin C (ascorbic acid), and 7 wt % of gluconolactone.
Comparative Example 8 (CE8): a composition containing 0.5 wt % of sodium ascorbyl phosphate, and 7 wt % of gluconolactone.
Comparative Example 9 (CE9): a composition containing 0.5 wt % of ascorbyl glucoside, and 7 wt % of gluconolactone.
Inventive Example 3 (IE3): a composition containing 0.5 wt % of 3-ethyl ascorbic acid, and 7 wt % of gluconolactone.
Comparative Example 10 (CE10): a composition containing 1 wt % of vitamin C (ascorbic acid), and 7 wt % of gluconolactone.
Comparative Example 11 (CE11): a composition containing 1 wt % of sodium ascorbyl phosphate, and 7 wt % of gluconolactone.
Comparative Example 12 (CE12): a composition containing 1 wt % of ascorbyl glucoside, and 7 wt % of gluconolactone.
Inventive Example 4 (IE4): a composition containing 1 wt % of 3-ethyl ascorbic acid, and 7 wt % of gluconolactone.

### Testing

The examples are evaluated for stability by analysis of colour changes over time using a X-Rite Color™ i7 Benchtop Spectrophotometer with transmission kit for liquid measurements. The test procedure involves the following steps for each new sample: (1) Calibrate spectrophotometer for transmission measurement, (2) Fill cuvette with test solution, (3) Insert cuvette into instrument, (4) Measure initial (standard) L* a* b* values for each example, and (5) Clean and dry solution for next measurement.

After initial measurement each example is placed in a humidity cabinet at 40 °C. The changes in colour are measured for each example after 1, 7 and 14 days after each sample is stored in a cabinet at 40 °C. Prior to colour change measurements each sample is allowed to cool to 23 °C. Each example is then evaluated by the following steps 1-5 above.

The DE2000 rating after initial measurement in the spectrophotometer is normalised to be 0. The results obtained after 14 days for each example are shown in the following table. Typically better results are obtained for examples having a lower DE2000 rating (DE2000 is unitless measure of colour change). An increase in DE2000 indicates an example is becoming less stable over time.

| Example | DE2000 | Example | DE2000 |
|---|---|---|---|
| CE1 | 4.7 | CE7 | 22.0 |
| CE2 | 0.28 | CE8 | 0.61 |
| CE3 | 0.22 | CE9 | 0.39 |
| IE1 | 0.05 | IE3 | 0.07 |
| | | | |
| CE4 | 9.46 | CE10 | 7.27 |
| CE5 | 2.32 | CE11 | 2.71 |
| CE6 | 0.77 | CE12 | 1.88 |
| IE2 | 0.15 | IE4 | 0.09 |

The results obtained indicate that the composition of the disclosed technology has improved stability compared to comparative examples containing Vitamin C, or sodium ascorbyl phosphate, or ascorbyl glucoside.

## Claims

1. A cosmetic composition comprising:
0.001 to 10 wt % of an O-substituted ascorbic acid represented by the formula: 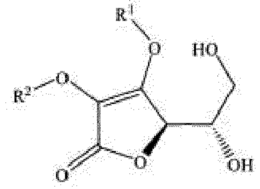 wherein R¹ is C1-20 alkyl or C3-20 cycloalkyl and R² is H; and
0.01 to 10 wt % gluconolactone,
wherein the composition comprises an aqueous phase present at up to 95 wt %, or up to 90 wt % of the composition.

2. The cosmetic composition of claim 1 comprising 0.01 to 3 wt % of O-substituted ascorbic acid and 0.1 to 2.5 wt % of gluconolactone or derivative thereof.

3. The cosmetic composition of claim 2 comprising 0.1 to 2 wt % of O-substituted ascorbic acid and 0.5 to 2 wt % of gluconolactone, or derivatives thereof.

4. The cosmetic composition of any preceding claim, wherein the composition is an oil-in-water emulsion comprising 30 to 85 wt % of an aqueous phase, and 15 to 70 wt % of an oil phase.

5. The cosmetic composition of claim 4, wherein the emulsion comprises 60 to 75 wt % of an aqueous phase and 25 to 40 wt % of an oil phase.

6. The cosmetic composition of any preceding claim, wherein the O-substituted ascorbic acid is 3-ethyl ascorbic acid.

7. The cosmetic composition of any preceding claim, wherein the composition is in the form of a gel, cream, lotion or serum.

8. The cosmetic use of a cosmetic composition of any preceding claim.

9. The cosmetic use of claim 8, wherein the cosmetic composition is a skin care composition.

10. A method of improving skin care comprising supplying to skin a cosmetic composition of any one of claims 1 to 7.

11. The method of claim 10, wherein improving skin care includes improving (i) skin exfoliation, or moisturisation, or (ii) decreasing or preventing at least one of the following, forming wrinkles or fine lines, skin sagging, or hyperpigmentation, or (iii) increasing skin firmness or skin laxity.

12. The use of the cosmetic composition of any one of claims 1 to 7 to improve skin condition.

## Patentansprüche

1. Kosmetische Zusammensetzung, umfassend:
0,001 bis 10 Gew .-% O-substituierte Ascorbinsäure, dargestellt durch die folgende Formel: 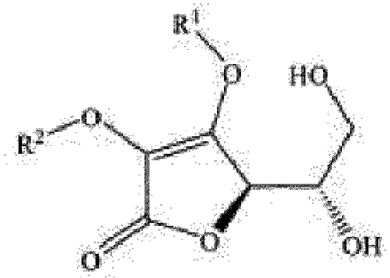 wobei R¹ C1-20 Alkyl oder C3-20 Cycloalkyl und R² H ist; und
0,01 bis 10 Gew.-% Gluconolacton,
wobei die Zusammensetzung eine wässrige Phase umfasst, die zu bis zu 95 Gew .-% oder zu bis zu 90 Gew .-% der Zusammensetzung vorliegt.

2. Kosmetische Zusammensetzung nach Anspruch 1, umfassend 0,01 bis 3 Gew.-% O-substituierte Ascorbinsäure und 0,1 bis 2,5 Gew .-% Gluconolacton oder Derivat davon.

3. Kosmetische Zusammensetzung nach Anspruch 2, umfassend 0,1 bis 2 Gew.-% O-substituierte Ascorbinsäure und 0,5 bis 2 Gew.-% Gluconolacton oder Derivate davon.

4. Kosmetische Zusammensetzung nach einem der vorhergehenden Ansprüche, wobei die Zusammensetzung eine Öl-in-Wasser-Emulsion ist, die 30 bis 85 Gew.-% einer wässrigen Phase und 15 bis 70 Gew .-% einer Ölphase umfasst.

5. Kosmetische Zusammensetzung nach Anspruch 4, wobei die Emulsion 60 bis 75 Gew .-% einer wässrigen Phase und 25 bis 40 Gew .-% einer Ölphase umfasst.

6. Kosmetische Zusammensetzung nach einem der vorhergehenden Ansprüche, wobei die O-substituierte Ascorbinsäure 3-Ethylascorbinsäure ist.

7. Kosmetische Zusammensetzung nach einem der vorhergehenden Ansprüche, wobei die Zusammensetzung in Form eines Gels, einer Creme, einer Lotion oder eines Serums vorliegt.

8. Kosmetische Verwendung einer kosmetischen Zusammensetzung nach einem der vorhergehenden Ansprüche.

9. Kosmetische Verwendung nach Anspruch 8, wobei die kosmetische Zusammensetzung eine Hautpflegezusammensetzung ist.

10. Verfahren zur Verbesserung der Hautpflege, umfassend das Zuführen einer kosmetischen Zusammensetzung nach einem der Ansprüche 1 bis 7 zur Haut.

11. Verfahren nach Anspruch 10, wobei das Verbessern der Hautpflege das Verbessern (i) des Hautpeelings oder der Befeuchtung oder (ii) das Verringern oder Verhindern von mindestens einem der Folgenden, das Bilden von Falten oder feinen Linien, das Absacken der Haut oder die Hyperpigmentierung oder (iii) Erhöhung der Hautfestigkeit oder Hautschlaffheit umfasst.

12. Verwendung der kosmetischen Zusammensetzung nach einem der Ansprüche 1 bis 7 zur Verbesserung des Hautzustands.

## Revendications

1. Composition cosmétique comprenant :
0,001 à 10 % en poids d'un acide ascorbique O-substitué représenté par la formule : 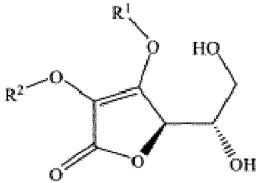 dans laquelle R¹ est un alkyle en C₁ à C₂₀ ou un cycloalkyle en C₃ à C₂₀ et R² est H ; et
0,01 à 10 % en poids de gluconolactone,
laquelle composition comprend une phase aqueuse présente à raison de jusqu'à 95 % en poids ou jusqu'à 90 % en poids de la composition.

2. Composition cosmétique selon la revendication 1, comprenant 0,01 à 3 % en poids d'acide ascorbique O-substitué et 0,1 à 2,5 % en poids de gluconolactone ou d'un dérivé de celle-ci.

3. Composition cosmétique selon la revendication 2, comprenant 0,1 à 2 % en poids d'acide ascorbique O-substitué et 0,5 à 2 % en poids de gluconolactone ou de dérivés de celle-ci.

4. Composition cosmétique selon l'une quelconque des revendications précédentes, laquelle composition est une émulsion huile dans l'eau comprenant 30 à 85 % en poids d'une phase aqueuse et 15 à 70 % en poids d'une phase huileuse.

5. Composition cosmétique selon la revendication 4, dans laquelle l'émulsion comprend 60 à 75 % en poids d'une phase aqueuse et 25 à 40 % en poids d'une phase huileuse.

6. Composition cosmétique selon l'une quelconque des revendications précédentes, dans laquelle l'acide ascorbique O-substitué est l'acide 3-éthylascorbique.

7. Composition cosmétique selon l'une quelconque des revendications précédentes, laquelle composition est sous la forme d'un gel, d'une crème, d'une lotion ou d'un sérum.

8. Utilisation cosmétique d'une composition de l'une quelconque des revendications précédentes.

9. Utilisation cosmétique selon la revendication 8, dans laquelle la composition cosmétique est une composition de soin de la peau.

10. Procédé pour améliorer les soins de la peau, comprenant la délivrance à la peau d'une composition cosmétique de l'une quelconque des revendications 1 à 7.

11. Procédé selon la revendication 10, dans lequel l'amélioration des soins de la peau comprend une amélioration (i) de l'exfoliation ou de l'hydratation de la peau, ou (ii) une diminution ou une prévention d'au moins l'une des suivantes, la formation de rides ou de ridules, l'affaissement de la peau, ou une hyperpigmentation, ou (iii) une augmentation de la fermeté de la peau ou du relâchement de la peau.

12. Utilisation de la composition cosmétique de l'une quelconque des revendications 1 à 7 pour améliorer l'état de la peau.
